# EUROPÄISCHE PATENTSCHRIFT

(11) **EP 3 065 676 B1**
(45) Veröffentlichungstag und Bekanntmachung des Hinweises auf die Patenterteilung: **20.07.2022**
(21) Anmeldenummer: 14809598.7
(22) Anmeldetag: 04.11.2014
(51) Int. Cl.: A61F 2/90, A61F 2/954, A61F 2/958, A61F 2/82

(54) **STENT MIT RÜCKHALTEELEMENT**
STENT COMPRISING A RETAINING ELEMENT
STENT AVEC ÉLÉMENT DE RETENUE

(30) Priorität: 04.11.2013 DE 102013018426
(43) Veröffentlichungstag der Anmeldung: 14.09.2016
(73) Patentinhaber: Bentley InnoMed GmbH, 72379 Hechingen (DE)
(72) Erfinder: KASPAR, Klaus, A-8010 Graz (AT)
(74) Vertreter: Hoefer & Partner Patentanwälte mbB
(86) Internationale Anmeldenummer: PCT/EP2014/073635
(87) Internationale Veröffentlichungsnummer: WO 2015/063312

(56) Entgegenhaltungen:
- EP-A1- 1 870 057
- WO-A2-2007/104051
- WO-A2-2008/111069
- US-A1- 2002 091 434
- US-A1- 2011 060 398

## Beschreibung

Die Erfindung betrifft einen Stent zur Positionierung in der Aufzweigung eines Blutgefäßes, wobei der Stent eine Vielzahl von Maschen, ein proximales Ende und ein distales Ende aufweist.

Im Stand der Technik sind verschiedene Einführsysteme führ Stents, beispielsweise Flechtstents oder ballonexpandierbare Stents, zum Positionieren in einer vorbestimmten Position in der Aufzweigung eines Blutgefäßes bekannt. Die Positionierung ist jedoch mit herkömmlichen Methoden problematisch; selbst in einfachen T-Gabelungen und V-Gabelungen haben sich beim Positionieren der Stents Komplikationen ergeben. Insbesondere treten solche Komplikationen dann auf, wenn der Stent mit seinem Ende in das Stammgefäß, von der der Abzweig ausgeht, hineinragt. In diesem Fall können sich Thromben bilden, die zu Gefäßverschlüssen führen können. Auch können in das Stammgefäß hineinragende Enden eines Stents, der in der Abzweigung platziert ist, dass Stenten des Stammgefäßes erschweren. Insbesondere ergeben sich Komplikationen bei einer Aufzweigung in Form einer V-Gablung mit einem Winkel von weniger als 70° zwischen den beiden Armen.

Aus der WO 2007/104051 A2 ist ein Maschenstent bekannt, bei dem ein Führungsdraht durch eine Masche des Stents geführt ist.

Damit besteht ein Bedürfnis, Einführsysteme für Stents in Gefäßaufzweigungen zu verbessern, um eine größere Positionierungsgenauigkeit des Stents am Platzierungsort zu erzielen.

Zur Lösung der Aufgabe wird erfindungsgemäß ein Stent der eingangs genannten Art mit einem Rückhaltelement versehen, das durch die gegenüberliegenden Maschen am proximalen Ende des Stents geführt ist.

Bevorzugte Ausgestaltungsformen der Erfindung sind in den Unteransprüchen angegeben, die jeweils einzeln oder in Kombination einen Aspekt der Erfindung darstellen können.

Erfindungsgemäß ist der Stent zur Positionierung in der Aufzweigung eines Gefäßes ein weithin üblicher Stent mit einem proximalen und einem distalen Ende und einer Vielzahl von Maschen oder Zellen, bei dem das Rückhalteelement durch gegenüberliegende Maschen am proximalen Ende des Stents geführt ist. Zweckmäßiger ist das Rückhalteelement mit einem Führungsdraht verbunden, in einer bevorzugten Ausführungsform das distale Ende eines Führungsdrahts, das von außen durch gegenüberliegende Maschen des Stents an dessen proximalen Ende hindurch gezogen ist. Das Rückhalteelement ist entfernbar und wird nach Platzierung des Stents aus dem Stent und dem Blutgefäß entfernt. Insbesondere besteht das Rückhalteelement aus Draht, kann jedoch aus einem Kunststoff bestehen, der eine hinreichende Steifigkeit aufweist. Bevorzugte Materialien für das Rückhalteelement sind medizinischer Stahl und Nitinol.

Es versteht sich, dass der hier verwandte Begriff proximal das Ende bezeichnet, dass zum behandelnden Arzt hinweist und der Begriff distal das vom behandelnden Arzt abgewandte Ende des Führungsdrahts oder Stents.

Der Begriff Stent bezeichnet ein medizinisches Implantat, das in ein Gefäß eingebracht wird, um dies zu erweitern oder offen zu halten. Es handelt sich zum Beispiel um einen Gittergerüst in Röhrchen Form aus Metall oder Kunststoff, wobei der Stent aus einem entsprechenden Rohr geschnitten sein kann, etwa durch Laserschneiden, oder aber auch geflochten sein kann. Materialien für den Stent sind übliche Materialien, wobei bei selbstexpandierenden Stents ein Formgedächtnismaterial eingesetzt wird, etwa Nitinol oder Federstahl, bei ballonexpandierbaren Stents ein für diese Zwecke üblicher medizinischer Stahl.

Der Begriff Maschen oder Zellen beschreibt die im Gittergerüst des Stents vorhandenen Öffnungen. Gegenüberliegende Maschen oder Zellen sind an der Peripherie des Stents einander zugewandte Öffnungen auf gegenüberliegenden Seiten.

Bei der Anwendung wird der erfindungsgemäße Stent mit dem Rückhalteelement auf übliche Art und Weise durch einen Katheter an seinen Einsatzort geführt, in der Regel aufgekrimpt auf einen Ballon, mit dem die spätere Expansion durchgeführt wird. Das Rückhalteelement ist vormontiert und wird mit dem Stent durch den Katheter an den Einsatzort geführt. Handelt es sich bei dem Rückhalteelement um einen einfachen Draht, beispielsweise das Ende eines Führungsdrahts, liegt dieser in dem Katheter in doppelt abgewinkelter Form vor, wobei das abgewinkelte Endenach proximal aufweist. Nach dem Freisetzen aus dem Katheter, d. h. wenn der Stent seinen Einsatzort erreicht hat, spreizt sich das Rückhalteelement auf und legt sich vor das abzweigende Gefäß dergestalt, dass der Stent mit seinem proximalen Ende präzise an den Anfang des abzweigenden Gefäßes platziert wird. Nach der Expansion mit Hilfe des Ballons oder der Selbstexpansion wird das Rückhalteelement, hier in Form eines Führungsdrahts, zurückgezogen; der Stent hat seine endgültige und exakt vorgesehene Stellung erreicht.

Gemäß einer weiteren bevorzugten Ausführungsform hat das Rückhalteelement in seinem distalen Bereich eine T-Form mit zwei Armen, die von Ihnen durch gegenüberliegende Maschen des Stents geführt sind. Der Querbalken der T-Form bildet dabei die Rückhaltevorrichtung, der Stiel des T ist mit dem Führungsdraht verbunden, der damit am proximalen Ende des Stents endet allenfalls um ein Weniges in das Innere des Stents hineinragt.

Auch in dieser Ausführungsform sind die aus dem Stent herausragenden freien Enden des Rückhalteelements nach proximal abgewinkelt und werden in dieser Form durch einen Katheter geführt. Nach Rückziehen des Katheters - wenn das Stent seine Position erreicht hat - spreizen sich die freien Enden auf, legen sich vor das abgehende Gefäß und fixieren den Stent in der Position, in der er zur Expansion kommt oder gebracht wird.

Zweckmäßigerweise kann die Katheterfunktion auch durch einen rückziehbaren Schlauch ausgeübt werden, in den der Stent mit dem Rückhalteelement und zugehörigen Führungsdraht, gegebenenfalls auch der Expandiervorrichtung, geführt ist. Der Schlauch ist rückziehbar und wird unmittelbar nach der Platzierung des Stents abgezogen, sodass sich die freien Enden des Rückhalteelements abspreizen können.

Um die notwendige Flexibilität des Rückhalteelements zu gewährleisten, besteht dies zweckmäßigerweise aus einem Federstahl oder einer Nickel-TitanLegierung mit Formgedächtniseigenschaften (Nitinol).

Es versteht sich, dass Stent und Rückhalteelement erfindungsgemäß eine Einheit bilden und im vormontierten Zustand vorliegen. Entsprechendes gilt für ein Kit aus auf einem Ballon aufgekrimmten Stent, Rückhalteelement mit Führungsdraht, gegebenenfalls dem Rückhalteschlauch sowie dem Platzierungskatheter.

Es versteht sich, dass als Stent ein jeder Stent verwandt werden kann, der die entsprechenden Durchbrechungen in Form einer Zell- oder Maschenstruktur aufweist. Entsprechend kann jeder herkömmliche Stent mit dieser Struktur erfindungsgemäß modifiziert und mit dem Rückhalteelement ausgestattet werden. Dies erlaubt eine weite Auswahl an Stents, die in einer Gefäßabzweigung implantiert werden können und dabei den Vorgaben der Gefäßstruktur Rechnung tragen. Das Prinzip des "Sperrriegels" an der Gefäßabzweigung durch das oder die freien Enden des Rückhalteelements, dass die punktgenaue Platzierung des Stents ermöglicht, bleibt stets das gleiche.

Es versteht sich ferner, dass der Stent an die jeweilige Form der Abzeigung angepasst werden kann, insbesondere am proximalen Ende schräg verlaufen kann.

Nachfolgend wird die Erfindung anhand der anliegenden Abbildungen exemplarisch erläutert, wobei die im Einzelnen dargestellten Merkmale sowohl einzeln als auch in beliebiger Kombination einen Aspekt der Erfindung darstellen. Es zeigen:
- Fig. 1: eine schematische Ansicht eines Einführsystems mit einem erfindungsgemäßen Stent in ein Gefäß nach einer ersten Ausführungsform;
- Fig. 2: eine schematische Ansicht des Einführsystems nach Fig.1 beim positionieren des Stents in einer Aufzweigung;
- Fig. 3: eine schematische Ansicht des Einführsystems nach Fig.1 nach Positionierung des Stents in der vorbestimmten Position;
- Fig. 4: eine Ansicht des Stents in seiner vorbestimmten Position in der Aufzweigung eines Gefäßmodells;
- Fig. 5: eine schematische Ansicht eines Einführsystems mit einem erfindungsgemäßen Stent in ein Gefäß nach einer zweiten Ausführungsform;
- Fig. 6: eine Ansicht des Einführsystems nach Anspruch 6 beim Positionieren des Stents an seiner vorbestimmten Position in der Aufzweigung eines Gefäßmodells;
- Fig. 7: eine Ansicht des Stents in seiner Endposition.

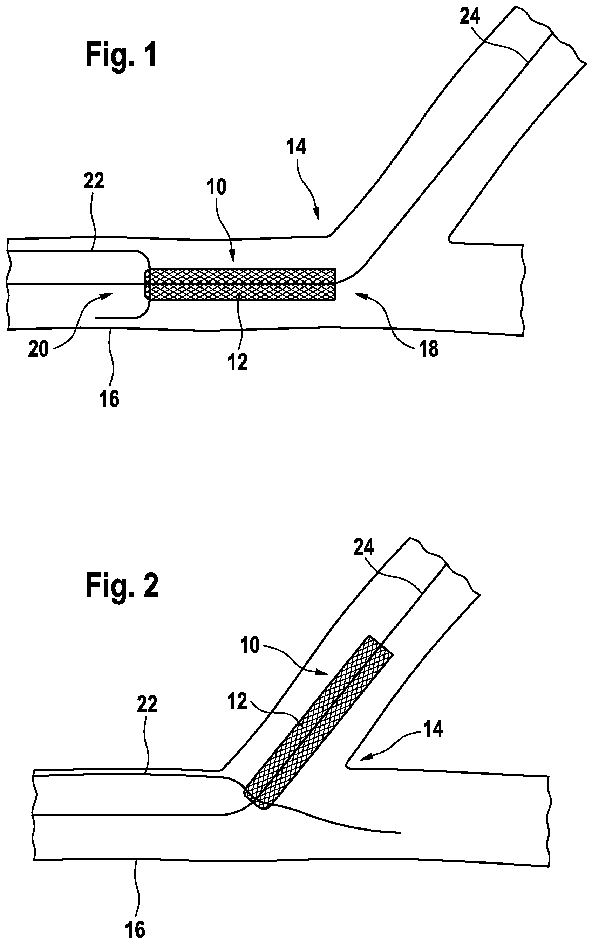

Figur 1 zeigt das System zur Positionierung eines Stents 12 in der Aufzweigung 14 eines Blutgefäßes in diesem Fall einer V-Gabelung. Der Stent 12 weist eine Vielzahl von Maschen 10, am distalen Ende 18 an einem proximalen Ende 20 auf. Um den Stent in einer vorbestimmten Position in der Aufzweigung 14 des Gefäßes 16 zu halten ist am proximalen Ende 20 des Stents von außen durch gegenüberliegende Maschen 10 ein Rückhalteelement 22 durchgeführt, in diesem Fall das distale Ende 22 eines konventionellen Führungsdrahts. Zur Festlegung am Stent 12 ist das distale Ende 22 des Führungsdrahts von außen durch zwei einander gegenüberliegenden Maschen 10 am proximalen Ende 20 des Stents 12 geführt dergestalt, dass das freie Ende des Führungsdraht seitlich aus dem Stent herausragt. Während des Transports sind der Führungsdraht und das freie Ende abgewinkelt, insbesondere durch Transport in einem herkömmlichen Katheter, nach der Freisetzung des Stents aus dem Katheter spreizt sich das distale Ende 22 des Führungsdrahts auf und legt sich vor die Aufzweigung 14 des Gefäßes 16.

Bei dem Stent handelt es sich in der Regel um einen ballonexpandierbaren Stent, der nach der Platzierung mit Hilfe des Ballons hydraulisch expandiert und an den Gefäßwänden festgelegt wird. Nach der Festlegung an der Gefäßwand wird das Rückhalteelement 22 nicht mehr benötigt und aus seiner Position am proximalen Ende 20 des Stents 12 herausgezogen und entfernt. Die Ballonexpansion des Stents erfolgt nach üblichen Verfahren mit üblichen Mitteln.

Nicht dargestellt in Figur 1 ist, dass das Rückhalteelement 22 und zumindest das proximale Ende 20 des Stents 12 in einem Schlauch angeordnet sein kann, der zur Freigabe des Stents und des Rückhalteelements abgezogen werden kann.

Gemäß Figur 1 wird der Stent 12 mit Hilfe eines üblichen Führungsdrahts, aufgekrimpt auf einen üblichen Ballon (nicht dargestellt), bis zu seiner vorbestimmten Position in der Aufzweigung 14 des Gefäßes 16 geführt. Dargestellt ist der Führungsdraht 24, der der Platzierung des Stents dient. Weiterhin ist das Rückhalteelement 22 derart durch den Stent 12 geführt, dass das Endstück über den Stent hinausragt. In der Einführphase des Stents 12 bildet das Rückhalteelement 22 im Katheter im Wesentlichen ein U aus, dass sich nach Erreichen der Position und Freisetzen des Stents zu dem Sperrriegel streckt, siehe Figur 2.

In Figur 2 ist der nächste Schritt der Platzierung des Stents 12 in der Aufzweigung 14 eines Gefäßesystems dargestellt. Das freie Ende des Rückhalteelements 22 ist abgespreizt und legt sich für die Mündung des abzweigenden Gefäßes, wodurch der Stent am weiteren Eindringen in den Abzweig gehindert ist. In dieser vorbestimmten Position in der Aufzweigung 14 wird dann durch den Ballon (nicht dargestellt) expandiert und festgelegt.

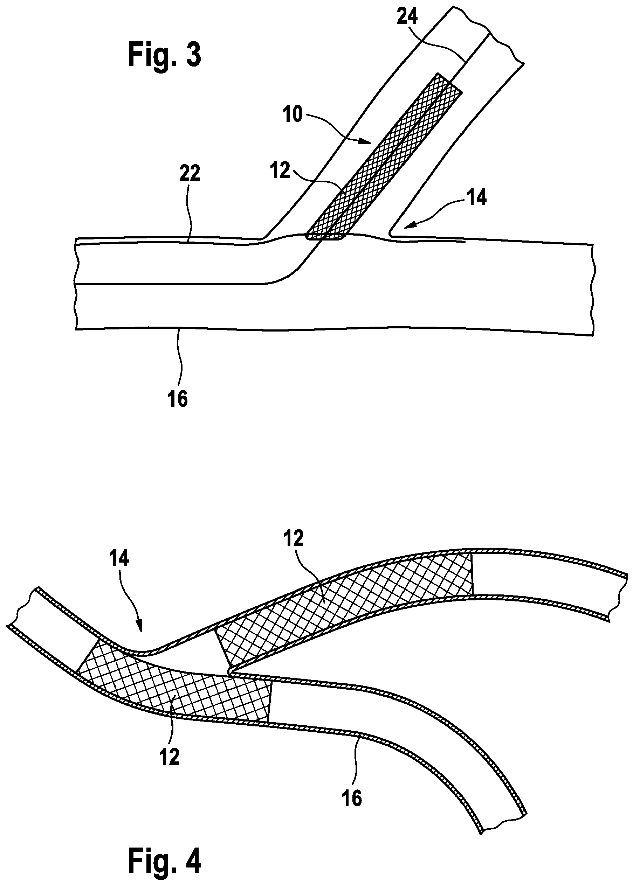

Figur 3 zeigt dann den Stent 12 in seiner Endposition unmittelbar vor der Expansion. Deutlich ist zu sehen, dass das Rückhalteelement 22 den Stent in seiner Stellung fixiert. Ballon und Katheter für die Expansion sind nicht dargestellt. Im dargestellten Fall ist das proximale Ende des Stents schräg ausgebildet, um eine optimale Anpassung an den Verlauf des abzweigenden Gefäßes zu erzielen.

Figur 4 zeigt ein Stent 12 in der Aufzweigung 14 eines Gefäßsystems 16. nachdem der Stent 12 seine vorbestimmte Positionierung in der Aufzweigung 14 erreicht hat, wurde er mit Hilfe eines Ballons aufgeblasen. Danach werden das Rückhalteelement 22, der Führungsdraht 24, Ballon und Katheter entfernt.

In Figur 4 ist weiterhin erkennbar, dass in das Stammgefäß in Höhe der Abzweigung ein weiterer Stent 12 positioniert wurde.

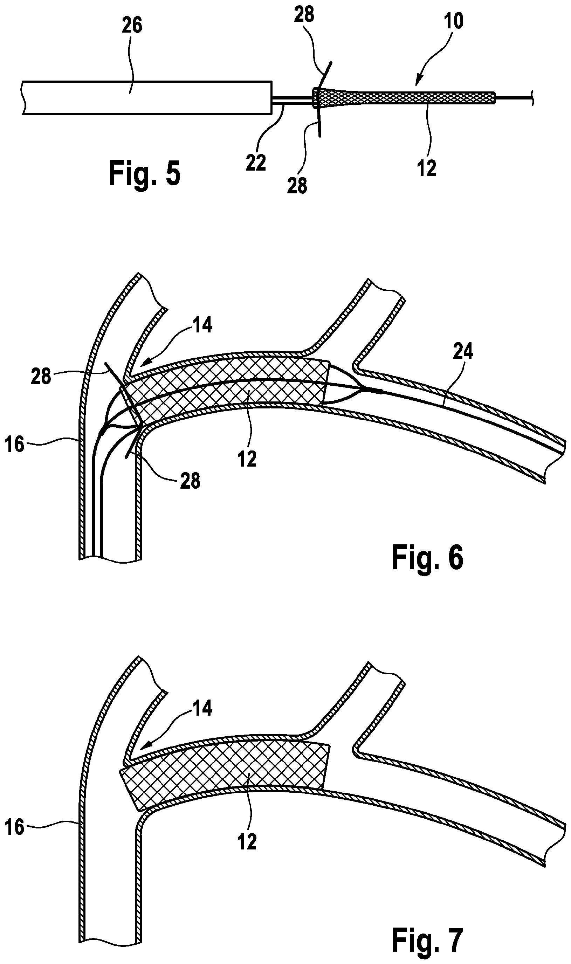

In Figur 5 ist eine weitere Ausführungsform der Erfindung dargestellt. In dieser Ausführungsform ist das Rückhalteelement 22 in das proximale Ende 20 des Stents 12 hineingeführt und ragt mit zwei Armen 28 seitlich durch zwei Maschen aus dem Stent heraus. Die Maschen, durch die die beiden Arme hinausragen, liegen einander gegenüber. Die Arme bilden zusammen mit dem Stent und dem distalen Abschnitt 22 des Führungsdrahts ein T. Weiterhin weist das System einen Schlauch 26 auf, der den Stent 12 und die Arme 28 des Rückhalteelements 22 während des Transports durch das Gefäßsystem umschließt. Der Schlauch 26 wird bei der Platzierung zurückgezogen; Figur 5 zeigt den Schlauch in bereits zurückgegebenen Zustand, durch den die Arme 28 des Rückhalteelements 22 freigegeben sind und auspreizen.

Figur 6 zeigt, wie der Stent 12 mit Hilfe eines Führungsdrahts 24 bis zu seiner vorbestimmten Position in der Aufzweigung 14 geführt wird und in seiner Position durch die aufgespreizten Arme 28 des Rückhalteelements 22 fixiert wird. Nach der Expansion (Figur 7) wird das Rückhalteelement 22, der Führungsdraht 24 und der für die Expansion benutzte Ballon zurückgezogen. Der Schlauch ist bereits vorher abgezogen worden.

Es versteht sich, dass die in den vorstehenden Abbildungen dargestellten Stents auch selbst expandierend sein können, d.h. zur Expansion keines Ballons bedürfen.

## Patentansprüche

1. Eine Einheit umfassend einen Stent zur Positionierung in der Aufzweigung (14) eines Blutgefäßes (16), wobei der Stent (12) eine Vielzahl von Maschen (10), ein distales (18) und ein proximales Ende (20) aufweist, und ein entfernbares Rückhalteelement, **dadurch gekennzeichnet, dass** das Rückhalteelement (22) ein Führungsdraht ist, der mit seinem distalen Ende (22) von außen durch zwei einander gegenüberliegende Maschen (10) aus einer Mehrzahl von Maschen am proximalen Ende (20) des Stents (12) geführt ist und sich das Rückhalteelement (22) am Einsatzort des Stents aufspreizt, und sich nach dem Sperrriegel-Prinzip vor das abzweigende Gefäß dergestalt legt, dass der Stent mit seinem proximalen Ende präzise an den Anfang des abzweigenden Gefäßes platziert wird.

2. Einheit nach Anspruch 1, **dadurch gekennzeichnet, dass** das Rückhalteelement (22) aus einem Formgedächtnismaterial, vorzugsweise Nitinol besteht.

3. Einheit nach einem der vorstehenden Ansprüche, **dadurch gekennzeichnet, dass** Stent (12) und Rückhalteelement (20) von einem rückziehbaren Schlauch (26) umgeben sind, dergestalt, dass das oder die freien Enden des Rückhalteelements (22) im Schlauch anliegen und nach Abziehen des Schlauchs (26) abspreizen können.

4. Einheit nach einem der Ansprüche 1 bis 3, **dadurch gekennzeichnet, dass** der Stent ballonexpandierbar ist.

5. Kit, bestehend aus einem Katheter und einer Einheit nach Anspruch 4.

## Claims

1. A unit comprising a stent to be positioned in the bifurcation/branching (14) of a blood vessel (16), wherein the stent (12) comprises of a plurality of meshes (10) and has a distal (18) and a proximal end (20), and a removable retaining element, **characterized in that** said retaining element (22) is a guidewire, the distal end of said guidewire being guided from outside of the stent through two oppositely arranged meshes (10) selected from a plurality of meshes situated at the proximal end (20) of the stent (12) and wherein at the target position of the stent, the retaining element (22) spreads out, and assumes a position in front of the opening of the vessel branch to form a locking bar in such a way that the proximal end of the stent is placed precisely at the position where the vessel is branching off.

2. Unit according to claim 1, **characterized in that** the retaining element (22) consists of a shape-memory material, preferably of Nitinol.

3. Unit according to any one of the preceding claims, **characterized in that** the stent (12) and the retaining element (20) are enclosed within a retractable tube (26) in such a manner that the free end or the free ends of the retaining element (22) are enfolded within the tube and are capable of spreading out once the tube (26) is withdrawn.

4. Unit according to any one of claims 1 to 3, **characterized in that** the stent is balloon-expandable.

5. Kit consisting of a catheter and a unit according to claim 4.

## Revendications

1. Unité comprenant un stent à positionner dans la ramification (14) d'un vaisseau sanguin (16), dans laquelle le stent (12) présente une pluralité de mailles (10), une extrémité distale (18) et une proximale (20), et un élément de retenue pouvant être retiré, **caractérisée en ce que** l'élément de retenue (22) est un fil de guidage, qui est guidé avec son extrémité distale (22) de l'extérieur à travers deux mailles (10) opposées l'une à l'autre parmi une pluralité de mailles sur l'extrémité proximale (20) du stent (12) et l'élément de retenue (22) s'écarte sur le lieu d'utilisation du stent, et se pose devant le vaisseau se ramifiant selon le principe du verrou de fermeture, de telle sorte que le stent est placé avec son extrémité proximale de manière précise au début du vaisseau se ramifiant.

2. Unité selon la revendication 1, **caractérisée en ce que** l'élément de retenue (22) est constitué d'un matériau à mémoire de forme, de préférence du nitinol.

3. Unité selon l'une quelconque des revendications précédentes, **caractérisée en ce que** le stent (12) et l'élément de retenue (20) sont entourés par un tuyau (26) rétractable, de telle sorte que la ou les extrémités libres de l'élément de retenue (22) s'appliquent dans le tuyau et peuvent s'écarter après le retrait du tuyau (26).

4. Unité selon l'une quelconque des revendications 1 à 3, **caractérisée en ce que** le stent est expansible par ballon.

5. Kit, constitué d'un cathéter et d'une unité selon la revendication 4.
